# EUROPÄISCHE PATENTSCHRIFT

(11) **EP 2 352 017 B1**
(45) Veröffentlichungstag und Bekanntmachung des Hinweises auf die Patenterteilung: **31.07.2013**
(21) Anmeldenummer: 11000172.4
(22) Anmeldetag: 12.10.2009
(51) Int. Cl.: G01N 27/22, G01N 33/36, G01R 27/26

(54) **Verfahren zum Testen einer kapazitiven Messvorrichtung**
Method for testing a capacitive measuring device
Procédé pour tester un dispositif de mesure capacitaire

(30) Priorität: 16.10.2008 CH 16462008
(43) Veröffentlichungstag der Anmeldung: 03.08.2011
(62) Teilanmeldung aus: 09748935.5
(73) Patentinhaber: Uster Technologies AG, 8610 Uster (CH)
(72) Erfinder: Gehrig, Reto, 8406 Winterthur (CH)

(56) Entgegenhaltungen:
- EP-A2- 1 124 134
- WO-A2-2004/083813
- DE-A1- 19 535 177
- DE-A1- 19 850 290
- GB-A- 1 061 635
- US-A- 3 684 954
- US-A- 3 757 211
- US-A- 4 208 625
- US-A- 4 706 203
- US-A- 4 772 844

## Beschreibung

### FACHGEBIET

Die vorliegende Erfindung liegt auf dem Gebiet der elektrischen Messvorrichtungen. Sie betrifft ein Verfahren zum Testen einer Vorrichtung oder zum Testen von einer Vorrichtung nachgeschalteten Komponenten, wobei die genannte Vorrichtung zur Bestimmung mindestens einer dielektrischen Eigenschaft einer Kondensatoranordnung dient.

Ein bevorzugtes Einsatzgebiet für die Erfmdung ist die kapazitive Prüfung von länglichen, vorzugsweise textilen Gebilden wie Kardenband, Vorgarn, Garn oder Gewebe. Eine derartige Prüfung kann bspw. die Detektion von Fremdstoffen oder das Erkennen von Änderungen der Masse pro Längeneinheit und/oder die Messung von Feuchtigkeit im Prüfgut zum Ziel haben. Die Erfindung kann bspw. im Produktionsprozess (online) in Garnreinigern auf Spinn- oder Spulmaschinen oder in der Laborprüfung (offline) in Garnprüfgeräten eingesetzt werden.

### STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden verschiedene bekannte Sensorprinzipien; hier interessiert besonders das kapazitive Messprinzip. Bei diesem ist ein Messkondensator typischerweise als ebener Plattenkondensator ausgeführt und weist eine Durchgangsöffnung für das Prüfgut auf. Der Messkondensator ist Teil eines LC-Oszillators, so dass bei Anregung des LC-Oszillators eine elektrische Wechselspannung am Messkondensator anliegt. Die Durchgangsöffnung wird dadurch mit einem elektrischen Wechselfeld beaufschlagt. Das Prüfgut wird durch den Plattenkondensator hindurch bewegt und dem Wechselfeld ausgesetzt. Es wird ein elektrisches Ausgangssignal des Plattenkondensators detektiert. In einer Auswerteschaltung werden aus dem Ausgangssignal dielektrische Eigenschaften des Prüfgutes ermittelt. Aus den dielektrischen Eigenschaften werden Parameter des Prüfgutes wie Masse pro Längeneinheit und/oder Materialzusammensetzung bestimmt. Ein kapazitiver Garn- oder Bandsensor ist z. B. in der GB-638,365 A beschrieben.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Vorrichtung nebst dem eigentlichen Messkondensator einen Referenzkondensator. Dieser kann durch Zufügen einer dritten, parallel zu den beiden Messkondensatorplatten angeordneten Kondensatorplatte gebildet werden, wobei die drei Kondensatorplatten zu einer kapazitiven Messschaltung zusammen geschaltet werden. Beispiele für Messschaltungen und geeignete Auswerteschaltungen für deren Ausgangssignale finden sich in den Schriften Es-0'924'513 A1, WO-2006/105676 A1 und WO-2007/115416 A1.

Aus der US-4,843,879 A ist ein Gerät für die kapazitive Qualitätskontrolle von textilen Fäden bekannt. Es beinhaltet eine Doppelkondensatoranordnung mit einem Messkondensator und einem Referenzkondensator. Die Doppelkondensatoranordnung ist in einem elektrischen Schaltkreis eingebaut. Der Schaltkreis beinhaltet einen Oszillator zum Anlegen zweier Wechselspannungen mit entgegengesetzten Phasen an die beiden äusseren Kondensatorelektroden der Doppelkondensatoranordnung. In jedem Ast zwischen dem Oszillator und der jeweiligen Elektrode befinden sich ein Signalverstärker und ein Abgleichkondensator. Die Abgleichkondensatoren dienen dem Abgleich des

Ausgangssignals der Doppelkondensatoranordnung ohne Prüfgut auf den Wert Null.

Ein weiteres kapazitives Garnprüfgerät, umfassend einen Messkondensator und einen elektronisch steuerbaren Abgleichkondensator, ist aus DE 19535177 bekannt.

Die US-4,208,625 A offenbart em Verfahren zur automatischen Justierung einer Messvorrichtung mit einer Kapazitätsmessbrücke für Filamente. Die Justierung dient dazu, einen Nullpunkt und eine Verstärkung der Kapazitätsmessbrücke für den bestimmungsgemässen Betrieb einzustellen. Ein Impuls auf einer Ausgangsleitung der Kapazitätsmessbrücke aktiviert ein Schieberegister. Danach steuert das Schieberegister die automatische Justierung. Es schliesst unter anderem einen Schalter, der einen Widerstand an einen Arm der Kapazitätsmessbrücke anschliesst, um das Vorhandensein eines Filamentes zu simulieren. Das konstante Ausgangssignal der Kapazitätsmessbrücke wird mit einer grösser werdenden Verstärkung multipliziert, bis es einen vorgegebenen Wert erreicht hat. Die so ermittelte Verstärkung wird gespeichert und auf nachfolgende Messungen angewendet, um eine Verstärkungsdrift der Kapazitätsmessbrücke zu kompensieren.

### DARSTELLUNG DER ERFINDUNG

Es ist eine Aufgabe der vorliegenden Erfindung, ein Verfahren zum Testen der Messvorrichtung oder zum Testen von der Messvorrichtung nachgeschalteten Komponenten anzugeben.

Diese und andere Aufgaben werden durch das erfindungsgemässe Verfahren, wie es im unabhängigen Patentanspruch definiert ist, gelöst. Vorteilhafte Ausführungsformen sind in den abhängigen Patentansprüchen angegeben.

Die Erfindung beruht auf der Idee, Abgleichmittel in einem elektrischen Pfad zwischen einem Wechselsignalgenerator und einer die Kondensatoranordnung beinhaltenden Messschaltung vorzusehen, die mittels eines elektrischen Steuersignals steuerbar sind, um ein Ausgangssignal der Vorrichtung abzugleichen. Mit solchen externen Abgleichmitteln kann die Vorrichtung abgeglichen werden, ohne in die Kondensatoranordnung selbst einzugreifen. Dies eröffnet nicht nur die Möglichkeit eines automatischen Abgleichs der Vorrichtung, sondern auch zur Einspeisung von beliebigen elektrischen Steuersignalen in die Vorrichtung.

Unter dem Begriff "Kondensatoranordnung" wird in dieser Schrift eine Anordnung mit zwei Körpern, die vom elektrischen Wechselsignal des Wechselsignalgenerators ungleichartig aufladbar und durch mindestens ein Dielektrikum voneinander getrennt sind, verstanden. In einer bevorzugten Ausführungsform handelt es sich bei der Kondensatoranordnung um einen Kondensator mit zwei voneinander beabstandeten Platten, zwischen denen sich Luft befindet und zwischen die ein zu untersuchendes bewegtes längliches textiles Prüfgut einführbar ist. Unter dem Begriff "elektrisches Wechselsignal" wird in dieser Schrift ein elektrisches Spannungs- oder Stromsignal mit mindestens einem sich zeitlich ändernden, vorzugsweise periodischen Anteil (AC-Anteil) verstanden, dem zusätzlich ein zeitlich im Wesentlichen konstanter Anteil (DC-Anteil, Offset) überlagert sein kann.

Die Vorrichtung zur Bestimmung mindestens einer dielektrischen Eigenschaft einer Kondensatoranordnung beinhaltet mindestens einen Wechselsignalgenerator zum Anlegen eines elektrischen Wechselsignals an die Kondensatoranordnung. Sie beinhaltet ferner eine Auswerteschaltung zur Auswertung mindestens einer elektrischen Messgrösse eines an der Kondensatoranordnung abgegriffenen elektrischen Signals. Ausserdem beinhaltet die Vorrichtung Abgleichmittel, die in einem elektrischen Pfad zwischen dem mindestens einen Wechselsignalgenerator und der Kondensatoranordnung angeordnet sind und mittels derer mindestens ein Parameter des elektrischen Wechselsignals derart veränderbar ist, dass ein Ausgangssignal der Auswerteschaltung bei definierten, konstanten Bedingungen einen bestimmten Wert, vorzugsweise Null, annimmt. Es sind Steuermittel zur Abgabe eines elektrischen Steuersignals an die Abgleichmittel vorhanden, mittels dessen die Veränderung des mindestens einen Parameters steuerbar ist.

Bevorzugt weist die Vorrichtung eine Rückkopplung auf, mittels deren ein Ausgangssignal der Kondensatoranordnung oder der Auswerteschaltung auf die Steuermittel einwirkt. Die Kondensatoranordnung ist vorzugsweise vom Wechselsignalgenerator derart abgekoppelt, dass sie die Grundfrequenz und die Signalform des angelegten Wechselsignals nicht wesentlich beeinflusst.

Zur Realisierung der Abgleichmittel werden verschiedene beispielhafte Varianten vorgeschlagen, wobei die Aufzählung nicht abschliessend ist:
- Die Abgleichmittel beinhalten eine Mehrzahl von elektrischen Widerständen, die einzeln oder gruppenweise zu- oder wegschaltbar sind.
- Die Abgleichmittel beinhalten einen Modulator für eine Amplitudenmodulation des elektrischen Wechselsignals.
- Die Abgleichmittel beinhalten einen Verstärker mit variabler oder programmierbarer Verstärkung zur Verstärkung des elektrischen Wechselsignals.
- Die Abgleichmittel beinhalten ein digitales Potenziometer oder einen Rejustor.
- Die Abgleichmittel beinhalten eine Kapazitätsdiode.

Die Vorrichtung beinhaltet vorzugsweise einen Referenzkondensator, welcher in Serie zur Kondensatoranordnung geschaltet ist. Dabei kann der mindestens eine Wechselsignalgenerator dazu eingerichtet sein, an die Kondensatoranordnung bzw. an den Referenzkondensator zwei elektrische Wechselspannungen mit entgegengesetzten Phasen anzulegen. Die Abgleichmittel können im elektrischen Pfad zwischen dem Wechselsignalgenerator und der Kondensatoranordnung und/oder im elektrischen Pfad zwischen dem mindestens einen Wechselsignalgenerator und dem Referenzkondensator angeordnet sein.

Eine bevorzugte Verwendung der Vorrichtung liegt in der kapazitiven Untersuchung eines bewegten länglichen textilen Prüfgutes wie Kardenband, Vorgarn, Garn oder Gewebe, wobei das bewegte Prüfgut die Kondensatoranordnung beeinflusst.

Im erfindungsgemässen Verfahren wird die Kondensatoranordnung im Wesentlichen zeitlich unverändert belassen, und das elektrische Steuersignal ist von einem Ausgangssignal der Kondensatoranordnung unabhängig. Dabei kann das elektrische Steuersignal ein sich zeitlich schnell änderndes, synthetisch erzeugtes und/oder vorgängig gespeichertes Signal sein. Das erfindungsgemässe Verfahren kann zum Testen der erfindungsgemässen Vorrichtung oder zum Testen von der erfindungsgemässen Vorrichtung nachgeschalteten Komponenten angewendet werden.

Als unmittelbare Vorteile, welche die Vorrichtung bietet, können unter anderen folgende genannt werden:
- Erstens braucht der Abgleich nicht in der Messschaltung zu erfolgen. Die Messschaltung ist der wohl empfindlichste Teil der kapazitiven Vorrichtung und ist somit äusserst empfindlich auf Störungen und Nichtlinearitäten. Indem sie Abgleichmittel ausserhalb der Messschaltung anordnet, vermeidet die Erfindung derartige Störungen in der Messschaltung.
- Zweitens kann erfindungsgemäss der Abgleich mit einer beliebigen Genauigkeit erfolgen. Es kann beispielsweise ein digitales Potenziometer eingesetzt werden, dessen Widerstand über eine Schnittstelle mit einer beliebig grossen Auflösung gesteuert werden kann.
- Drittens kann mit der Erfindung das Signal/Rauschverhältnis für all jene kapazitiven Sensoren merklich verbessert werden, bei welchen der Abgleich bisher durch einen trimmbaren Kondensator in der Messschaltung erfolgte. Dies deshalb, weil ein trimmbares Element in der Messschaltung dauernd eine Wirkung auf die Messschaltung ausübt.

Der Abgleich der Vorrichtung wird einfach, schnell und kostengünstig. Das Gerät muss nicht geöffnet werden, um die Messschaltung abzugleichen. Der Abgleich kann jederzeit erfolgen. Er kann automatisch vom Gerät selbst vorgenommen werden, ohne Eingriff einer Bedienungsperson. Dadurch wird es auch möglich, einen Abgleich zu irgendeinem beliebigen Zeitpunkt durchzuführen. So könnte ein Abgleich vor jeder Messung, vor jeder zehnten Messung, bei wesentlichen Änderungen von Langzeiteigenschaften des Ausgangssignals oder nach einer grösseren Änderung der Umgebungsbedingungen automatisch durchgeführt werden. Dies kann eine Erhöhung der Messgenauigkeit, der Messzuverlässigkeit, der Reproduzierbarkeit und allgemein eine Verbesserung der Messresultate zur Folge haben.

Die Abgleichmittel können aber nicht nur für das Abgleichen der Vorrichtung nützlich sein. Sie dienen erfindungsgemäss auch zur Einspeisung von beliebigen elektrischen Steuersignalen in die Vorrichtung. Mittels solcher Steuersignale wird die Vorrichtung gezielt verstimmt. Eine gezielte Verstimmung der Vorrichtung kann dieselbe oder eine ähnliche Wirkung haben wie eine Verstimmung durch eine Änderung der auszumessenden Kapazität. Durch Einspeisen von Steuersignalen in die Vorrichtung über die Abgleichmittel können also Messungen simuliert werden. Dabei sollten sich die Kapazitäten der Vorrichtung, mit Ausnahme der Abgleichmittel, nicht ändern. Simulierte Messungen können z. B. zum Testen einer Auswerteschaltung und zur Fehlersuche in der Auswerteschaltung verwendet werden. Sie können einem Abgleich der Auswerteschaltung, z. B. von in der Auswerteschaltung enthaltenen Filtern, dienen. Sie können auch zum Testen und/oder Einstellen der Vorrichtung dienen. Es ist möglich, vorgängig Ausgangssignale realer Messungen mit derselben Vorrichtung oder einem anderen Sensor aufzuzeichnen, in einem Speicher zu speichern und als Eingangssignale über die Abgleichmittel in die Vorrichtung einzuspeisen. Dadurch kann die Vorrichtung mit einer "Aufnahme und Wiedergabe"-Funktion ("record and playback") ausgestattet werden.

### AUFZÄHLUNG DER ZEICHNUNGEN

Nachfolgend wird die Erfindung anhand der schematischen Zeichnungen detailliert erläutert.
- Figur 1: zeigt ein Blockdiagramm der Vorrichtung.
- Figuren 2-9: zeigen Schaltschemata von verschiedenen Ausführungsformen der Vorrichtung.

### AUSFÜHRUNG DER ERFINDUNG

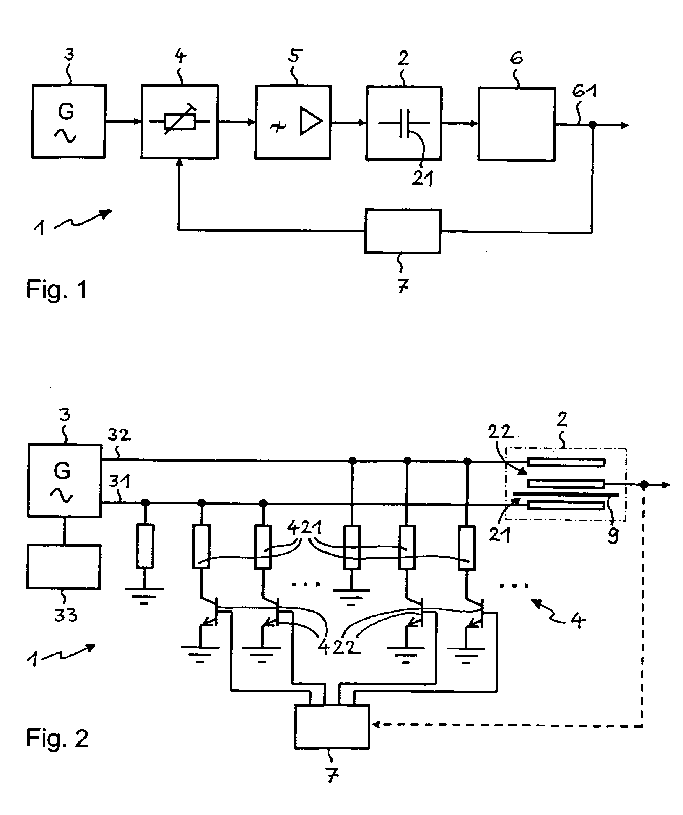

Figur 1 erklärt anhand eines einfachen Blockdiagramms die Vorrichtung 1. Diese dient zum Ausmessen einer Kapazität 21, welche in einer Messschaltung 2 eingesetzt ist. An die Messschaltung 2 wird ein elektrisches Wechselsignal, z. B. eine Wechselspannung, angelegt. Zur Erzeugung des Wechselsignals ist ein Wechselsignalgenerator 3 vorgesehen. Das vom Wechselsignalgenerator 3 erzeugte Wechselsignal kann noch in einer Filter- und/oder Verstärkerstufe 5 gefiltert und/oder verstärkt werden. Die Filter- und/oder Verstärkerstufe 5 dient vorzugsweise auch dazu, die auszumessende Kapazität 21 vom Wechselsignalgenerator 3 derart abzukoppeln, dass sie Parameter des vom Wechselsignalgenerator 3 erzeugten Wechselsignals, z. B. Frequenz, Phase und/oder Amplitude des Wechselsignals, nicht beeinflusst. Der Einfachheit halber ist die Filter- und/oder Verstärkerstufe 5 in den nachfolgenden Figuren nicht mehr eingezeichnet. Der Messschaltung 2 ist eine Auswerteschaltung 6 nachgeschaltet, die ein Ausgangssignal der Messschaltung 2 auswertet. Die Auswerteschaltung 6 kann als Rechner ausgebildet sein. Sie gibt ihrerseits auf einer Ausgangsleitung 61 ein Ausgangssignal aus, das ein Mass für die auszumessende Kapazität 21 ist. In der Vorrichtung 1 können selbstverständlich noch weitere Stufen vorgesehen sein. Insbesondere kann es vorteilhaft oder nötig sein, zwischen der Messschaltung 2 und der Auswerteschaltung 6 Filter, Verstärker und/oder Mischer einzubauen, die jedoch in den beiliegenden Zeichnungen der Einfachheit halber nicht dargestellt sind.

In einem elektrischen Pfad zwischen dem Wechselsignalgenerator 3 und der Messschaltung 2 sind Abgleichmittel 4 zum Abgleichen der Vorrichtung 1 angeordnet, mittels derer mindestens ein Parameter, z. B. die Amplitude, des elektrischen Wechselsignals veränderbar ist. Während des Abgleichs der Vorrichtung 1 sollte darauf geachtet werden, dass sich die auszumessende Kapazität 21 und allenfalls andere in der Messschaltung 1 vorhandene Kapazitäten zeitlich nicht ändern. Es sollten also zeitlich konstante Umgebungsbedingungen wie Temperatur und Luftfeuchtigkeit herrschen, oder es sollten entsprechende Änderungen kompensiert werden. Falls die auszumessende Kapazität 21 als Kondensator zur Aufnahme von Prüfgut ausgebildet ist (wie in den nachfolgenden Figuren dargestellt), sollte sich während des Abgleichs kein Prüfgut im Kondensator befinden, oder die Kapazität des Prüfgutes sollte sich zeitlich nicht ändern. Zum Abgleich der Vorrichtung 1 wird ein elektrisches Wechselspannungssignal an die auszumessende Kapazität 21 angelegt. Die Abgleichmittel 4 verändern mindestens einen Parameter des elektrischen Wechselsignals derart, dass das Ausgangssignal der Vorrichtung 1 auf der Ausgangsleitung 61 einen bestimmten Wert annimmt, vorzugsweise null wird. Zu diesem Zweck können die Abgleichmittel 4 manuell von einer Person eingestellt werden, bspw. nach der Herstellung oder beim Unterhalt der Vorrichtung 1. Alternativ können die Abgleichmittel 4 automatisch eingestellt werden. Die automatische Einstellung kann in einer speziell dafür vorgesehenen Steuereinheit 7 oder in der Auswerteeinheit 6 erfolgen. In Figur 1 ist eine Rückkopplung von der Ausgangsleitung 61 zu den Abgleichmitteln 4 eingezeichnet, die ein vom Ausgangssignal der Auswerteeinheit 6 bzw. der Messschaltung 1 abhängiges Steuersignal an die Abgleichmittel 4 abgibt. Mit einer automatischen Einstellung der Abgleichmittel 4 kann die Vorrichtung 1 automatisch abgeglichen werden. Somit liegt ein geschlossener Regelkreis vor, in dem das Ausgangssignal die Regelgrösse ist, die auf den Sollwert Null geregelt werden soll, die Steuereinheit 7 als Regler wirkt und das Steuersignal der Stellwert ist. Alternativ kann die Regelgrösse vor statt hinter der Auswerteeinheit 6 abgegriffen werden.

Eine erste Ausführungsform der Vorrichtung 1 ist in **Figur 2** dargestellt. Die Vorrichtung 1 dient zur kapazitiven Ausmessung eines Prüfgutes 9, z. B. eines Garns. Dazu wird das Prüfgut 9 in einen Messkondensator 21, z. B. einen ebenen Plattenkondensator, eingebracht. Der Messkondensator 21 bildet zusammen mit einem in Serie geschalteten Referenzkondensator 22 und möglicherweise weiteren (nicht eingezeichneten) Komponenten eine kapazitive Messschaltung 2. Der Messschaltung 2 kann, wie in Figur 1, eine Auswerteeinheit 6 nachgeschaltet sein, die jedoch in Figur 2 und in den nachfolgenden Figuren der Übersichtlichkeit halber nicht eingezeichnet ist.

Der Wechselsignalgenerator 3 kann z. B. ein Synthesizer, vorzugsweise ein direkter digitaler Synthesizer (direct digital synthesizer, DDS) sein. Der Synthesizer 3 kann von einer digitalen Schnittstelle 33 angesteuert werden. Der Synthesizer 3 hat vorzugsweise zwei Ausgänge 31, 32 für zwei elektrische Wechselsignale, die im Wesentlichen identisch, aber um 180° gegeneinander phasenverschoben sind. Dem Fachmann sind auch andere Wechselsignalgeneratoren bekannt, die zum Anlegen eines elektrischen Wechselsignals an die Messschaltung 2 geeignet sind, z. B. aus der folgenden Menge: RC-Oszillator, LC-Oszillator, Quarz-Oszillator, Oszillator mit Keramikresonator, Oszillator mit SAW-Komponente (akustische Oberflächenwellen, surface acoustic waves, SAW), Oszillator mit Logikbausteinen, Phasenregelschleife (phase-locked loop, PLL), Pulsweitenmodulator (pulse-width modulator, PWM), Kippstufe.

Im Ausführungsbeispiel von Figur 2 beinhalten die Abgleichmittel 4 eine Mehrzahl von parallel zueinander geschalteten Widerständen 421, die einzeln oder gruppenweise durch Schalter 422, z. B. Hochfrequenztransistoren, zu- und wegschaltbar sind. Dadurch ergibt sich ein veränderbarer Gesamtwiderstand, über welchem eine Spannung entsprechend abfällt, die zudem vom durch den Synthesizer abgegebenen Strom abhängt. Die Widerstände 421 können nur an einem der beiden Synthesizerausgänge oder, wie im Ausführungsbeispiel von Figur 1, an beiden Synthesizerausgängen 31, 32 angebracht sein. Die Schalter 422 können durch eine entsprechende digitale Schnittstelle 7 gesteuert werden.

Zum automatischen Abgleichen der Vorrichtung 1 kann eine Rückkopplung zwischen dem Ausgangssignal der Messschaltung 2 und der digitalen Schnittstelle 7, welche die Schalter 422 ansteuert, vorgesehen sein. Um die Vorrichtung abzugleichen, wird in einer bevorzugten Anwendung kein Prüfgut 9 in den Messkondensator eingerührt, und es wird auf zeitlich möglichst unveränderliche Umgebungsbedingungen wie Luftfeuchtigkeit und Temperatur geachtet. Mittels des Synthesizers 3 wird ein elektrisches Wechselsignal an die Messschaltung 2 angelegt, und die Stellung der Schalter 422 wird durch die digitale Schnittstelle 7 so geregelt, dass das Ausgangssignal der Messschaltung 2 null ist. Wenn dies der Fall ist, dann ist die Vorrichtung 1 abgeglichen, und die Stellung der Schalter 422 wird gespeichert und für die eigentlichen Messungen mit dem Prüfgut 9 beibehalten. Die digitale Schnittstelle 7 entspricht somit der Steuereinheit 7 von Figur 1 und wirkt als Regler in einem geschlossenen Regelkreis.

Die Möglichkeit der Rückkopplung und Regelung besteht in allen Ausführungsformen der Vorrichtung 1, ist aber der Einfachheit halber in den nachfolgenden Figuren nicht mehr eingezeichnet. Aus demselben Grund ist auch in den Figuren 2-9 die fakultative Filter- und/oder Verstärkerstufe 5 (siehe Figur 1) nicht eingezeichnet.

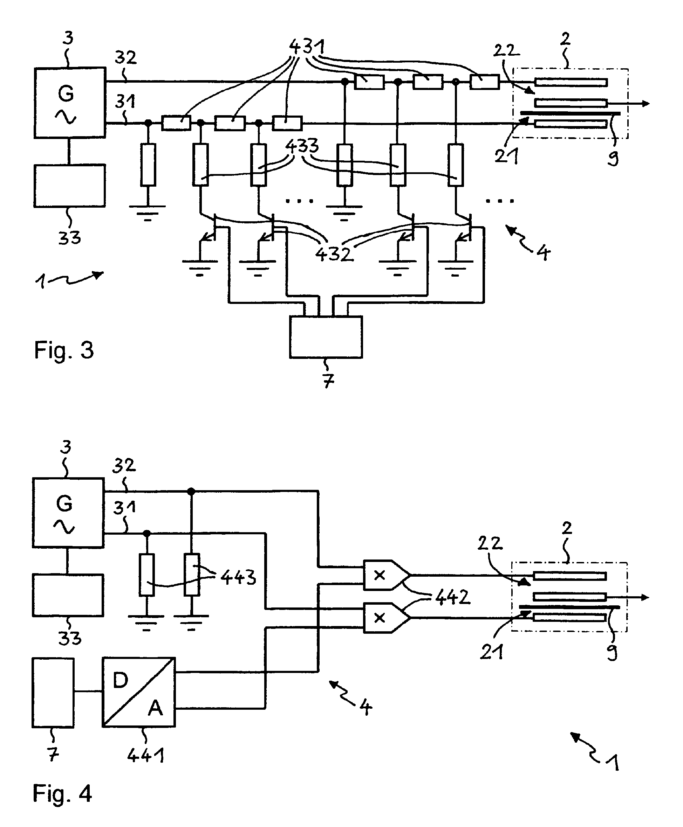

**Figur 3** zeigt eine zweite Ausführungsform der Vorrichtung 1. Hier sind die Abgleichmittel 4 als Widerstandsleitern ausgebildet. Eine solche Widerstandsleiter beinhaltet im Wesentlichen eine Mehrzahl von in Serie geschalteten Widerständen 431, die einzeln oder Gruppenweise durch Schalter 432 über weitere Widerstände 433 zu- oder weggeschaltet werden können. Durch geeignete Schalterstellungen, die wiederum von einer digitalen Schnittstelle 7 gesteuert werden können, kann ein Abgleich der Vorrichtung 1 erreicht werden.

In einer dritten Ausführungsform der Vorrichtung 1 gemäss **Figur 4** kommt ein differenzieller Digital/Analog-Wandler 441 mit zwei Ausgängen beliebiger Bit-Breite zum Einsatz. Der Digital/Analog-Wandler 441 wird von einer geeigneten digitalen Schnittstelle 7 gesteuert. Er erzeugt eine Spannung, die proportional zur Verstimmung bzw. zum Abgleich der Messschaltung 2 ist. Mit dieser Spannung wird in einem analogen Mischer oder Multiplizierer 442 die Wechselspannung des Synthesizers 3 moduliert, und das so modulierte Signal wird an die Messschaltung 2 angelegt. So kann die Amplitude der vom Synthesizer 3 erzeugten Wechselspannung beliebig eingestellt werden, bis die Vorrichtung 1 abgeglichen ist. Die beiden in Figur 4 eingezeichneten Widerstände 443 dienen der Wandlung eines vom Synthesizer 3 gelieferten Wechselstroms in eine Wechselspannung. Sie entfallen, falls der Synthesizer 3 bereits als Wechselspannungsquelle ausgebildet ist. Statt des differenziellen Digital/Analog-Wandlers 441 könnten zwei einpolige Digital/Analog-Wandler eingesetzt werden.

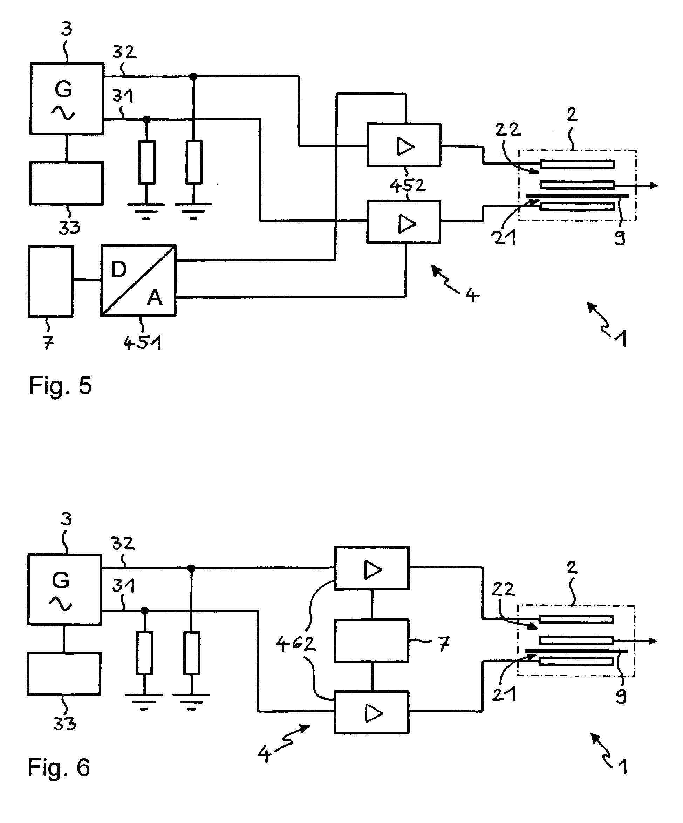

Eine vierte Ausführungsform der Vorrichtung 1, wie sie in **Figur 5** dargestellt ist, funktioniert nach dem Prinzip des spannungsgesteuerten Verstärkers. Sie beinhaltet zwei spannungsgesteuerte variable Verstärker 452 (variable gain amplifier, VGA) für die beiden Ausgangssignale des Synthesizers 3. Die Verstärkungen der variablen Verstärker 452 werden z. B. von einem Digital/Analog-Wandler 451 gesteuert, der seinerseits von einer digitalen Schnittstelle 7 gesteuert wird. Durch Einstellen von geeigneten Verstärkungen für die beiden Ausgangssignale des Synthesizers 3 kann ein Abgleich der Vorrichtung 1 erzielt werden.

Analog zur Ausführungsform von Figur 5 funktioniert eine fünfte Ausführungsform der Vorrichtung 1, die in **Figur 6** gezeigt ist. Statt der analog gesteuerten variablen Verstärker 452 kommen hier jedoch Verstärker 462 mit programmierbarer Verstärkung (programmable gain amplifier, PGA) zum Einsatz. Sie werden von einer geeigneten digitalen Schnittstelle 7 angesteuert. Der Digital/Analog-Wandler 451 entfällt bei dieser Ausführungsform.

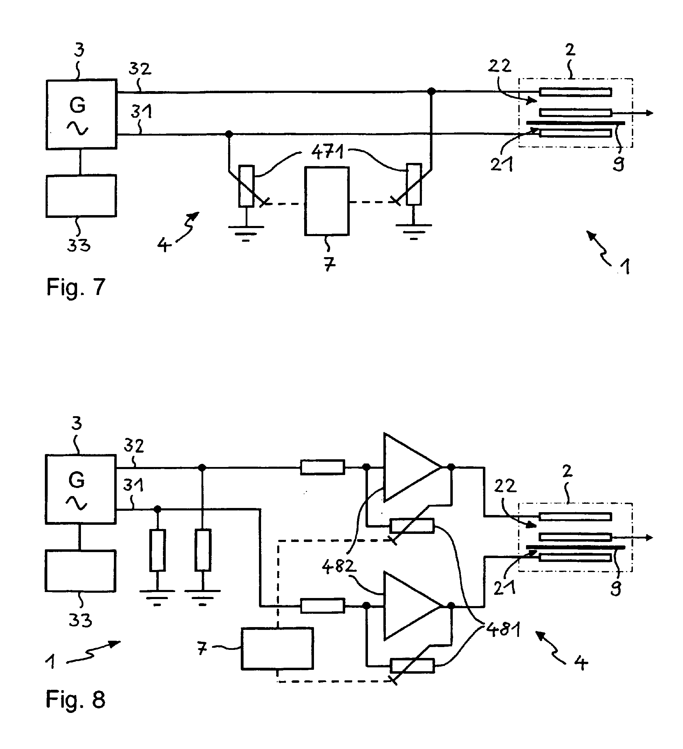

Eine sechste Ausführungsform der Vorrichtung 1 gemäss **Figur 7** verwendet digitale Potenziometer oder Rejustoren 471. Diese können über eine oder zwei digitale Schnittstellen 7 angesteuert werden, wodurch ihr Widerstand so verändert wird, dass ein gezielter Spannungsabfall durch den vom Synthesizer 3 erzeugten Wechselstrom entsteht. Falls der Synthesizer 3 als Wechselspannungsquelle ausgebildet ist, kann die Vorrichtung 1 alternativ als Spannungsteiler beschaltet sein.

Auch in einer siebten Ausführungsform der Vorrichtung 1, die in **Figur 8** dargestellt ist, werden digitale Potenziometer oder Rejustoren 481 eingesetzt, und zwar jeweils bspw. in der Gegenkopplung eines Verstärkers 482, welcher zwischen dem Synthesizer 3 und der Messschaltung 2 ein vom Synthesizer 3 erzeugtes Wechselsignal verstärkt.

Statt der digitalen Potenziometer 481 könnten in den Ausführungsformen der Figuren 7 und 8 alternativ herkömmliche, analoge Potenziometer eingesetzt werden. Mit ihnen könnte eine Bedienungsperson die Vorrichtung 1 manuell abgleichen.

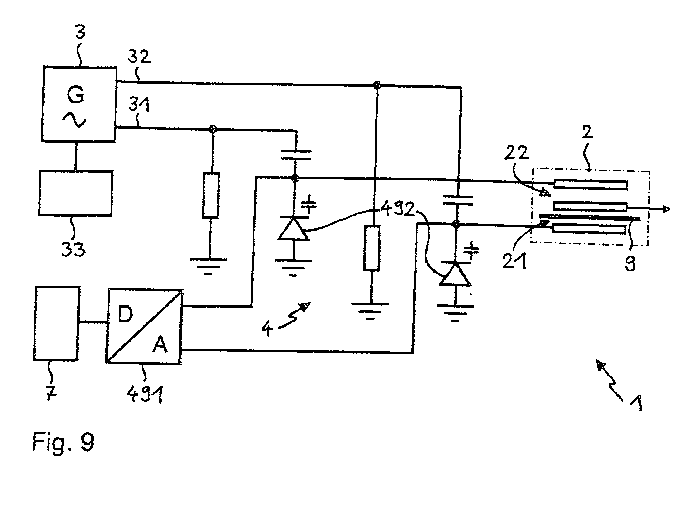

In einer achten Ausführungsform der Vorrichtung 1 gemäss **Figur 9** werden mit Kapazitätsdioden 492 kapazitive Spannungsteiler gebildet. Die Spannungen für die Steuerung der Kapazitätsdioden 492 werden z. B. von einem differenziellen Digital/Analog-Wandler 491 mit zwei Ausgängen geliefert, der seinerseits von einer digitalen Schnittstelle 7 gesteuert wird. Alternativ zum differenziellen Digital/AnalogWandler 491 könnten wiederum zwei einpolige Digital/Analog-Wandler eingesetzt werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Solche Varianten können z. B. Kombinationen der oben diskutierten Ausführungsformen sein. Dem Fachmann sind an sich viele elektrische Komponenten bekannt, die als Abgleichmittel 4 für die Vorrichtung 1 in Frage kommen.

### BEZUGSZEICHENLISTE

- 1: Vorrichtung

- 2: Messschaltung
- 21: auszumessende Kapazität, Messkondensator
- 22: Referenzkondensator

- 3: Wechselsignalgenerator
- 31,32: Ausgänge des Wechselsignalgenerators
- 33: digitale Schnittstelle zur Ansteuerung des Wechselsignalgenerators

- 4: Abgleichmittel
- 411: Digital/Analog-Wandler
- 421: Widerstände
- 422: Schalter
- 431, 433: Widerstände
- 432: Schalter
- 441: Digital/Analog-Wandler
- 442: Multiplizierer
- 451: Digital/Analog-Wandler
- 452: variabler Verstärker
- 462: Verstärker mit variabler Verstärkung
- 471: digitaler Potenziometer oder Rejustor
- 481: digitaler Potenziometer oder Rejustor
- 482: Verstärker
- 491: Digital/Analog-Wandler
- 492: Kapazitätsdiode

- 5: Filter- und/oder Verstärkerstufe

- 6: Auswerteschaltung

- 7: Steuereinheit

- 9: Prüfgut

## Patentansprüche

1. Verfahren zum Testen einer Vorrichtung (1) oder zum Testen von einer Vorrichtung (1) nachgeschalteten Komponenten, wobei
die genannte Vorrichtung (1) zur Bestimmung mindestens einer dielektrischen Eigenschaft einer Kondensatoranordnung (21) dient und
mindestens einen Wechselsignalgenerator (3) zum Anlegen eines elektrischen Wechselsignals an die Kondensatoranordnung (21),
eine Auswerteschaltung (6) zur Auswertung mindestens einer elektrischen Messgrösse eines an der Kondensatoranordnung (21) abgegriffenen elektrischen Signals,
Abgleichmittel (4), die in einem elektrischen Pfad zwischen dem mindestens einen Wechselsignalgenerator (3) und einer die Kondensatoranordnung (21) beinhaltenden Messschaltung (2) angeordnet sind und mittels derer mindestens ein Parameter des elektrischen Wechselsignals veränderbar ist, und Steuermittel (7) zur Abgabe eines elektrischen Steuersignals an die Abgleichmittel (4), mittels dessen die Veränderung des mindestens einen Parameters steuerbar ist,
beinhaltet,
wobei
die Kondensatoranordnung (21) im Wesentlichen zeitlich unverändert belassen wird, ein elektrisches Wechselsignal von dem mindestens einen Wechselsignalgenerator (3) erzeugt und an die Kondensatoranordnung (21) angelegt wird,
mindestens eine elektrische Messgrösse eines an der Kondensatoranordnung (21) abgegriffenen elektrischen Signals ausgewertet wird,
der mindestens eine Parameter des elektrischen Wechselsignals in dem elektrischen Pfad zwischen dem mindestens einen Wechselsignalgenerator (3) und der Messschaltung (2) durch die Abgleichmittel (4) verändert wird,
die Veränderung des mindestens einen Parameters mit dem von den Steuermitteln (7) abgegebenen elektrischen Steuersignal gesteuert wird, und
das elektrische Steuersignal von einem Ausgangssignal der Kondensatoranordnung (21) unabhängig ist.

2. Verfahren nach Anspruch 1, wobei das elektrische Steuersignal ein sich zeitlich schnell änderndes, synthetisch erzeugtes und/oder vorgängig gespeichertes Signal ist.

3. Verfahren nach einem der vorangehenden Ansprüche, wobei die Kondensatoranordnung (21) vom Wechselsignalgenerator (3) derart abgekoppelt ist, dass sie die Grundfrequenz und die Signalform des angelegten Wechselsignals nicht wesentlich beeinflusst.

4. Verfahren nach einem der vorangehenden Ansprüche, wobei die Abgleichmittel (4) eine Mehrzahl von elektrischen Widerständen (421, 431, 433) beinhalten, die einzeln oder gruppenweise zu- oder wegschaltbar sind.

5. Verfahren nach einem der vorangehenden Ansprüche, wobei die Abgleichmittel (4) einen Modulator (442) für eine Amplitudenmodulation des elektrischen Wechselsignals beinhalten.

6. Verfahren nach einem der vorangehenden Ansprüche, wobei die Abgleichmittel (4) einen Verstärker (452, 462) mit variabler oder programmierbarer Verstärkung zur Verstärkung des elektrischen Wechselsignals beinhalten.

7. Verfahren nach einem der vorangehenden Ansprüche, wobei die Abgleichmittel (4) ein digitales Potenziometer oder einen Rejustor (471,481) beinhalten.

8. Verfahren nach einem der vorangehenden Ansprüche, wobei die Abgleichmittel (4) eine Kapazitätsdiode (492) beinhalten.

9. Verfahren nach einem der vorangehenden Ansprüche, wobei die Vorrichtung (1) einen Referenzkondensator (22) beinhaltet, welcher in Serie zur Kondensatoranordnung (21) geschaltet ist.

10. Verfahren Anspruch 9, wobei der mindestens eine Wechselsignalgenerator (3) dazu eingerichtet ist, an die Kondensatoranordnung (21) bzw. an den Referenzkondensator (22) zwei elektrische Wechselspannungen mit entgegengesetzten Phasen anzulegen.

11. Verfahren nach Anspruch 10, wobei die Abgleichmittel (4) im elektrischen Pfad zwischen dem Wechselsignalgenerator (3) und der Kondensatoranordnung (21) und/oder im elektrischen Pfad zwischen dem mindestens einen Wechselsignalgenerator (3) und dem Referenzkondensator (22) angeordnet sind.

## Claims

1. A method for testing of an apparatus (1) or for testing of components connected downstream with an apparatus (1), wherein
said apparatus (1) is for determining at least one dielectric property of a capacitor arrangement (21) and contains
at least one alternating signal generator (3) for applying an electric alternating signal to the capacitor arrangement (21),
an evaluation circuit (6) for evaluating at least one electric measuring quantity of an electric signal tapped from the capacitor arrangement (21),
balancing means (4) which are arranged in an electric path between the at least one alternating signal generator (3) and a measuring circuit (2) containing the capacitor arrangement (21) and by means of which at least one parameter of the electric alternating signal is changeable, and
control means (7) for emitting an electric control signal to the balancing means (4), by means of which the change of the at least one parameter is controllable,
wherein
the capacitor arrangement (21) is essentially left in a temporally unchanged way, an electric alternating signal is generated by the at least one alternating signal generator (3) and is applied to the capacitor arrangement (21),
at least one electric measuring quantity of an electric signal tapped from the capacitor arrangement (21) is evaluated,
the at least one parameter of the electric alternating signal is changed in the electric path between the at least one alternating signal generator (3) and the measuring circuit (2) by the balancing means (4),
the change of the at least one parameter is controlled by the electric control signal emitted by the control means (7), and
the electric control signal is independent of an output signal of the capacitor arrangement (21).

2. The method according to claim 1, wherein the electric control signal is a temporally rapidly changing, synthetically generated and/or previously stored signal.

3. The method according to one of the preceding claims, wherein the capacitor arrangement (21) is decoupled from the alternating signal generator (3) in such a way that it does not relevantly influence the basic frequency and the signal shape of the applied alternating signal.

4. The method according to one of the preceding claims, wherein the balancing means (4) contain a plurality of electric resistors (421, 431, 433) which can be activated and deactivated individually or in groups.

5. The method according to one of the preceding claims, wherein the balancing means (4) contain a modulator (442) for an amplitude modulation of the electric alternating signal.

6. The method according to one of the preceding claims, wherein the balancing means (4) contain an amplifier (452, 462) with variable or programmable gain for amplifying the electric alternating signal.

7. The method according to one of the preceding claims, wherein the balancing means (4) contain a digital potentiometer or a rejustor (471, 481).

8. The method according to one of the preceding claims, wherein the balancing means (4) contain a variable-capacitance diode (492).

9. The method according to one of the preceding claims, wherein the apparatus (1) contains a reference capacitor (22) which is connected in series to the capacitor arrangement (21).

10. The method according to claim 9, wherein the at least one alternating signal generator (3) is arranged to apply two electric alternating voltages with opposite phases to the capacitor arrangement (21) and the reference capacitor (22), respectively.

11. The method according to claim 10, wherein the balancing means (4) are arranged in the electric path between the alternating signal generator (3) and the capacitor arrangement (21) and/or in the electric path between the at least one alternating signal generator (3) and the reference capacitor (22).

## Revendications

1. Procédé pour tester un dispositif (1) ou pour tester des composants montés en aval d'un dispositif (1), dans lequel
ledit dispositif (1) sert à déterminer au moins une propriété diélectrique d'une disposition de condensateurs (21) et comprend
au moins un générateur de signaux alternatifs (3) destiné à appliquer un signal alternatif électrique à la disposition de condensateurs (21),
un circuit d'analyse (6) destiné à analyser au moins une grandeur de mesure électrique d'un signal électrique capté au niveau de la disposition de condensateurs (21),
des moyens d'équilibrage (4) qui sont disposés dans un trajet électrique entre l'au moins un générateur de signaux alternatifs (3) et un circuit de mesure (2) incluant la disposition de condensateurs (21) et au moyen desquels un paramètre du signal alternatif électrique peut être modifié, et
des moyens de commande (7) destinés à émettre un signal de commande électrique vers les moyens d'équilibrage (4), au moyen duquel le changement d'un paramètre peut être commandé,
dans lequel
la disposition de condensateurs (21) est pour l'essentiel laissée inchangée dans le temps, un signal alternatif électrique est généré par l'au moins un générateur de signaux alternatifs (3) et appliqué à la disposition de condensateurs (21),
au moins une grandeur de mesure électrique d'un signal électrique capté au niveau de la disposition de condensateurs (21) est analysé,
l'au moins un paramètre du signal alternatif électrique est modifié dans le trajet électrique entre l'au moins un générateur de signaux alternatifs (3) et le circuit de mesure (2) par les moyens d'équilibrage (4),
le changement de l'au moins un paramètre est commandé avec le signal de commande électrique émis par les moyens de commande (7), et
le signal de commande électrique est indépendant d'un signal de sortie de la disposition de condensateurs (21).

2. Procédé selon la revendication 1, dans lequel le signal de commande électrique est un signal évoluant rapidement dans le temps, généré par synthèse et/ou enregistré au préalable.

3. Procédé selon l'une des revendications précédentes, dans lequel la disposition de condensateurs (21) est découplée du générateur de signaux alternatifs (3) de telle manière qu'elle n'influe pas notablement sur la fréquence fondamentale et la forme de signal du signal alternatif appliqué.

4. Procédé selon l'une des revendications précédentes, dans lequel les moyens d'équilibrage (4) incluent une pluralité de résistances électriques (421, 431, 433) qui peuvent être mises en circuit ou hors circuit individuellement ou par groupes

5. Procédé selon l'une des revendications précédentes, dans lequel les moyens d'équilibrage (4) incluent un modulateur (442) pour la modulation d'amplitude du signal alternatif électrique.

6. Procédé selon l'une des revendications précédentes, dans lequel les moyens d'équilibrage (4) incluent un amplificateur (452, 462) à amplification variable ou programmable pour l'amplification du signal alternatif électrique.

7. Procédé selon l'une des revendications précédentes, dans lequel les moyens d'équilibrage (4) incluent un potentiomètre numérique et une résistance variable (471, 481).

8. Procédé selon l'une des revendications précédentes, dans lequel les moyens d'équilibrage (4) incluent une diode à capacité variable (492).

9. Procédé selon l'une des revendications précédentes, dans lequel le dispositif (1) inclut un condensateur de référence (22) qui est monté en série avec la disposition de condensateurs (21).

10. Procédé selon la revendication 9, dans lequel l'au moins un générateur de signaux alternatifs (3) est équipé pour appliquer à la disposition de condensateurs (21) et au condensateur de référence (22) deux tensions alternatives électriques de phase opposée.

11. Procédé selon la revendication 10, dans lequel les moyens d'équilibrage (4) sont disposés dans le trajet électrique entre le générateur de signaux alternatifs (3) et la disposition de condensateurs (21) et/ou dans le trajet électrique entre l'au moins un générateur de signaux alternatifs (3) et le condensateur de référence (22).
